# EUROPEAN PATENT APPLICATION

(11) **EP 4 137 069 A1**
(43) Date of publication of application: **22.02.2023**
(21) Application number: 22190352.9
(22) Date of filing: 15.08.2022
(51) Int. Cl.: A61B 17/221

(54) **COMBINATION STENTRIEVER AND MICROCATHETER**

(30) Priority: 16.08.2021 US 202117402858
(71) Applicant: Neuravi Limited, Galway H91 K5YD (IE)
(72) Inventor: CONNOLLY, Patrick, Galway, H91 K5YD (IE)
(74) Representative: Carpmaels & Ransford LLP

(57) **Abstract**

A clot removal system for removing a clot from a body vessel, the clot removal system including: a microcatheter including: a distal segment comprising a first diameter; and a proximal segment comprising a second diameter; a stentriever positionable substantially within a lumen of a distal end of the microcatheter, the stentriever including: an engagement structure configured to expand when distal of the microcatheter in an expanded configuration, and an elongated member proximal of the engagement structure and extending to a proximal end of the microcatheter; and a hand control disposed on the proximal end of the microcatheter for controlling extending and retracting the engagement structure from the microcatheter.

## Description

### FIELD

The present application relates generally to clot retrieval devices, and specifically to combination stentriever and microcatheters.

### BACKGROUND

Deflectable or steerable catheters are used in various medical and surgical procedures, including ablation, such as arrhythmia ablation, mapping, such as cardiac mapping, and drug delivery, such as intracardial drug delivery. The steerable function can be accomplished by three modes of actions: straight translational movement along the direction of the catheter length; deflection of an end or distal section in one direction or in one plane; and turning of the catheter shaft to direct the deflected end toward the desired point. A control wire or pull wire positioned inside the catheter, usually connecting to the distal end, is used to direct the degree of deflection of the distal section. The degree of pulling on the mechanism directs the movement of the control wire and thus the degree of deflection of the distal end of the catheter shaft.

In the related art, the control wire is included within the catheter is a substantially straight lumen path. Thus, deflection is generally within one plane, having only a curl or sweep profile. operably connected to some type of a pulling mechanism, which is connected to a control device at the proximal end of the catheter shaft. This type of defection creates an "S" shape between a sheath and the catheter where the deflection mechanisms are in opposing directions.

Thus, catheters capable of improved control of the distal tip and those capable of forming a loop of variable sizes are desired in the art.

### SUMMARY

A clot removal system is presented herein which includes a clot removal system for removing a clot from a body vessel, the clot removal system. The clot removal system includes: a microcatheter including: a distal segment comprising a first diameter; and a proximal segment comprising a second diameter; a stentriever positionable substantially within a lumen of a distal end of the microcatheter, the stentriever including: an engagement structure configured to expand when distal of the microcatheter in an expanded configuration, and an elongated member proximal of the engagement structure and extending to a proximal end of the microcatheter; and a hand control disposed on the proximal end of the microcatheter for controlling extending and retracting the engagement structure from the microcatheter.

The microcatheter may be prevented from sliding proximal of the distal segment in a collapsed configuration.

The engagement structure may be configured to prevent the microcatheter from sliding proximal of the distal segment in a collapsed configuration.

The first diameter may be greater than the second diameter.

The hand control may include: a brace connected to the proximal end of the microcatheter; and a push-pull mechanism attached to the elongated member.

The push-pull mechanism may include a finger ring.

The hand control may include a lock to hold the engagement structure in a substantially fully retracted position.

The hand control may be single-handedly operable.

The microcatheter may further include a rapid exchange port extending from an outer surface of the microcatheter to the lumen, the rapid exchange port being dimensioned to receive a guidewire.

The engagement structure may be disposed within microcatheter proximal to the rapid exchange port.

The microcatheter may further include: a rapid exchange port on an outer surface of microcatheter, the rapid exchange port being dimensioned to receive a guidewire; and a guide lumen extending from the rapid exchange port to the distal end of the microcatheter.

The microcatheter may further include a rapid exchange port on an outer surface of the microcatheter. The elongated member may include a substantially hollow body extending through the engagement structure. The stentriever may further include a stentriever port extending from an outer surface of the elongated member to an interior of the hollow body, the rapid exchange port, hollow body, and stentriever port being dimensioned to receive a guidewire.

The stentriever port may be proximal the rapid exchange port.

The stentriever may further include a nose cone on a distal end of the engagement structure.

The lumen may include: a distal shaft extending proximally from the distal end, the distal shaft having a third diameter; and a proximal shaft extending distally from the proximal end, the proximal shaft having a fourth diameter less than the third diameter.

A diameter of the engagement structure in the expanded configuration may be greater than the third diameter such that the engagement structure cannot be fully retrieved within the distal shaft.

The microcatheter may further include: a proximal shaft extending distally from the proximal end; and a distal shaft extending distally from the proximal shaft, the engagement structure being disposed within the distal shaft and being unable to be received within the proximal shaft.

The microcatheter may further include: a proximal shaft extending distally from the proximal end, the lumen comprising a first diameter through the proximal shaft; and a distal shaft extending distally from the proximal shaft, the lumen comprising a second diameter through the distal shaft, the first diameter being less than the second diameter such that the engagement structure cannot be received by the lumen in the proximal shaft.

The hand control may be configured to control extending and retracting of the engagement structure from the distal end of the microcatheter.

The microcatheter may further include: a distal shaft extending proximally from the distal end; and a side port extending from an outer surface of the microcatheter at the distal shaft to the lumen. The engagement structure may be disposed within the distal shaft proximate the side port, the hand control is configured to control extending and retracting the engagement structure from the side port.

A distal end of the engagement structure may be attached to the microcatheter.

The engagement structure may include at least one scaffolding element to receive the clot and at least on pinching cell to pinch the clot.

The engagement structure may include: one or more pinching cells; and a shaped wire threaded through the one or more pinching cells.

The engagement structure may include a series of inner pinching cells and outer pinching cells.

The engagement structure may include: a structural thread; and a chain of pinching cells comprising a first end and a second end opposite each other, the first end and the second end of the chain being connected to the structural thread.

The engagement structure may include at least one stop configured to prevent full retraction of the engagement structure within the lumen after extension.

The at least one stop may prevent retraction of the engagement structure beyond a pinching configuration.

The hand control may further include a retrieval means to control withdrawing the at least one stop to enable full retraction of the engagement structure.

The clot removal system may further include: a first radioactive marker disposed on the distal end of the elongated member; and a second radioactive marker disposed on a distal end of the engagement structure.

According to aspects of the present disclosure, there is provided A method for removing a clot, the method including: operating a hand control to withdraw a microcatheter and extend an engagement structure of a stentriever from the microcatheter.

The method may further include embedding at least a portion of the engagement structure with at least a portion of the clot; and removing at least a portion of the clot by withdrawing the microcatheter and stentriever.

The method may further include operating the hand control to at least partially retract the engagement structure within the microcatheter prior to withdrawing the microcatheter and stentriever.

The engagement structure may move from a collapsed configuration within the microcatheter to an expanded configuration when the microcatheter is withdrawn.

The engagement structure may be prevented from sliding proximal of a distal segment of the microcatheter in the collapsed configuration.

The microcatheter may include: a distal segment comprising a first diameter; and a proximal segment comprising a second diameter, the first diameter being greater than the second diameter.

The hand control may include: a brace connected to a proximal end of the microcatheter; and a push-pull mechanism attached to the stentriever.

The push-pull mechanism may include a finger ring.

The method may further include operating the hand control to unlock a lock of the hand control prior to withdrawing the microcatheter, the lock holding the engagement structure in a substantially fully retracted position.

The hand control may be operated single-handedly.

The microcatheter may include a rapid exchange port extending from an outer surface of the microcatheter to a lumen of the microcatheter, the rapid exchange port being dimensioned to receive a guidewire.

The microcatheter may include: a rapid exchange port on an outer surface of microcatheter, the rapid exchange port being dimensioned to receive a guidewire; and a guide lumen extending from the rapid exchange port to a distal end of the microcatheter.

The microcatheter may further include a rapid exchange port on an outer surface of the microcatheter.

The stentriever may include: a substantially hollow body extending through the engagement structure; and a stentriever port extending from through the hollow body, the rapid exchange port, hollow body, and stentriever port being dimensioned to receive a guidewire.

The stentriever port may be proximal the rapid exchange port in a fully-retracted configuration.

The stentriever may include a nose cone on a distal end of the engagement structure.

The stentriever may be positioned within a lumen of the microcatheter. The lumen may include: a distal shaft extending proximally from a distal end of the microcatheter, the distal shaft having a third diameter; and a proximal shaft extending distally from a proximal end of the microcatheter, the proximal shaft having a fourth diameter less than the third diameter.

A diameter of the engagement structure in an expanded configuration may be greater than the third diameter such that the engagement structure cannot be fully retrieved within the distal shaft.

The microcatheter may include: a proximal end; a proximal shaft extending distally from the proximal end; and a distal shaft extending distally from the proximal shaft, the engagement structure being disposed within the distal shaft and being unable to be received within the proximal shaft.

The microcatheter may include: a lumen; a proximal end; a proximal shaft extending distally from the proximal end, the lumen comprising a first diameter through the proximal shaft; and a distal shaft extending distally from the proximal shaft, the lumen comprising a second diameter through the distal shaft, the first diameter being less than the second diameter such that the engagement structure cannot be received by the lumen in the proximal shaft.

The engagement structure may include at least one scaffolding element to receive the clot and at least on pinching cell to pinch the clot.

The engagement structure may include: one or more pinching cells; and a shaped wire threaded through the one or more pinching cells.

The engagement structure may include a series of inner pinching cells and outer pinching cells.

The engagement structure may include: a structural thread; and a chain of pinching cells comprising a first end and a second end opposite each other, the first end and the second end of the chain being connected to the structural thread.

The engagement structure may include at least one stop configured to prevent full retraction of the engagement structure within the microcatheter after extension.

The at least one stop may prevent retraction of the engagement structure beyond a pinching configuration.

The hand control further include a retrieval means to control withdrawing the at least one stop to enable full retraction of the engagement structure.

The microcatheter may include: a distal end; and a first radioactive marker disposed on the distal end of the microcatheter. The stentriever may include a second radioactive marker disposed on a distal end of the engagement structure.

### BRIEF DESCRIPTION OF THE DRAWINGS

While the specification concludes with claims, which particularly point out and distinctly claim the subject matter described herein, it is believed the subject matter will be better understood from the following description of certain examples taken in conjunction with the accompanying drawings, in which like reference numerals identify the same elements and in which:
Figure 1A illustrates a combination stentriever and microcatheter in a delivery configuration according to aspects of the present disclosure;
Figure 1B illustrates a combination stentriever and microcatheter in a deployed configuration according to aspects of the present disclosure;
Figure 1C illustrates a cross-sectional view of a distal portion of the combination stentriever and microcatheter of Figures 1A and 1B;
Figure ID illustrates a cross-sectional view of a proximal portion of the combination stentriever and microcatheter of Figures 1A and 1B;
Figures 2A-2B illustrate example hand controls for a combination stentriever and microcatheter according to aspects of the present disclosure;
Figures 3A-5 illustrate example combination stentriever and microcatheters according to aspects of the present disclosure;
Figures 6 and 7 illustrate example stentrievers according to aspects of the present disclosure;
Figures 8 illustrates production of a stentriever according to aspects of the present disclosure;
Figures 9A-10B illustrates combination stentriever and microcatheters with a side port according to aspects of the present disclosure;
Figures 11A and 11B illustrate an operation of a stentriever having a stop according to aspects of the present disclosure;
Figure 12 is a flowchart of a treatment incorporating an example clot removal device according to aspects of the present disclosure
Figure 13 is a flowchart of producing a combination stentriever and microcatheter according to aspects of the present disclosure;
Figure 14 illustrates a hand control with a lock according to aspects of the present disclosure;
Figure 15 illustrates an engagement structure having scaffolding elements according to aspects of the present disclosure; and
Figure 16 illustrates a combination stentriever and microcatheter with an engagement structure proximal to a rapid exchange port according to aspects of the present disclosure.

### DETAILED DESCRIPTION

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%.

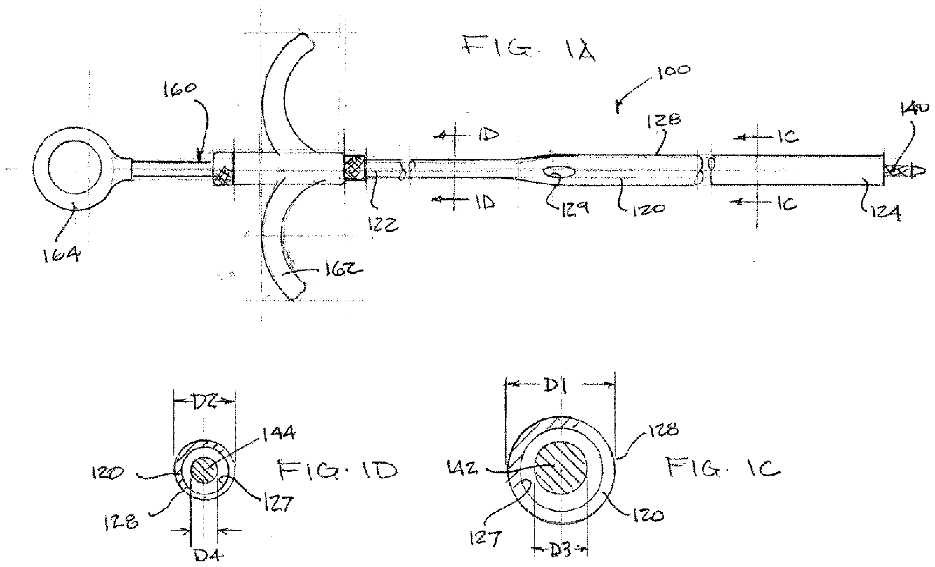

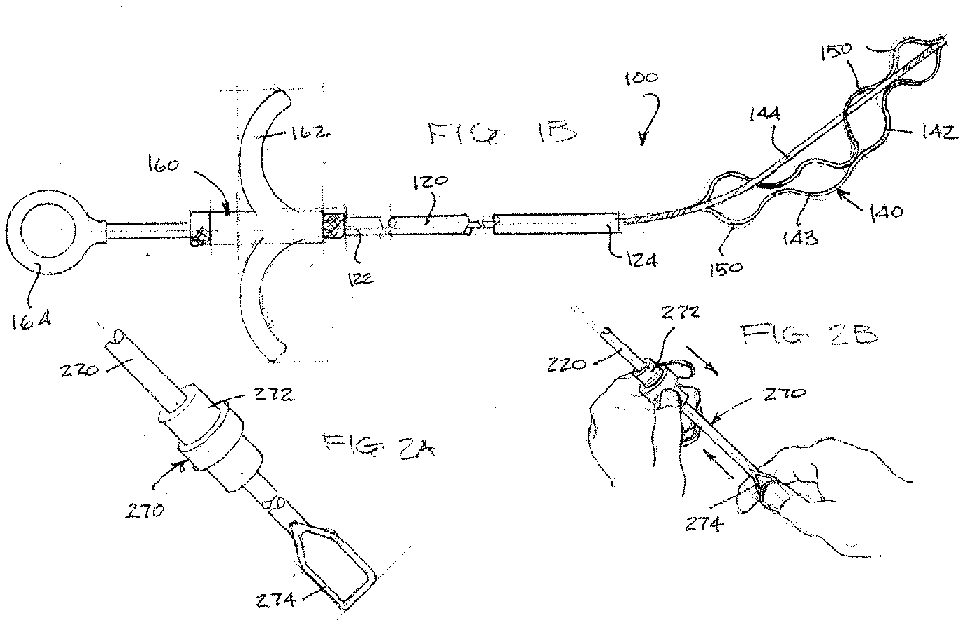

Figures 1A and 1B illustrate a combination stentriever and microcatheter 100 (e.g., a clot removal device) in delivery and deployed configurations, respectively. Figures 1C and ID illustrate cross-sectional views of distal and proximal portions of the combination stentriever and microcatheter 100, respectively. Combination stentriever and microcatheter 100 may include microcatheter 120, stentriever 140, and hand control 160. Microcatheter 120 has a proximal end 122 disposed near hand control 160, a distal end 124 disposed opposite thereof, and an outer surface 128. The a distal segment extending from the distal end 124 may have a first diameter D1, and a proximal segment extending from the proximal end 122 may have a second diameter D2. In some cases, the first diameter D1 may be greater than the second diameter D2. Microcatheter 120 includes a lumen 127 passing from the proximal end 122 to the distal end 124. The lumen 127 may have a distal shaft formed in the distal end 124 that has a third diameter D3 and a proximal shaft formed in the proximal end 126 that has a fourth diameter D4. The third diameter D3 may be greater than the fourth diameter D4. In some cases, the fourth diameter D4 may be dimensioned such that the engagement structure 142 cannot be received by the lumen 127 in a proximal portion of the microcatheter 120 (e.g., a proximal shaft). The catheter 120 may also have a rapid exchange port 129 extending from the outer surface 128 to the lumen 127. The rapid exchange port 129 may be dimensioned to receive the stentriever 140.

Stentriever 140 may include an engagement structure 142 and an elongated member 144 (e.g., a structural thread 144). The engagement structure 142 may include a chain 143 of pinching cells 150 including at least a first end and a second end pinching cell 150. The ends of the chain 143 can be connected to the structural thread 144. In some cases, in a delivery configuration, the engagement structure 142 may be disposed proximal the rapid exchange port 129 (i.e., nearer the hand control 160). In such a way, guidewire may avoid interfering with the stentriever 140. One of ordinary skill will recognize that the engagement structure 142 described herein is merely an example, and various additional or alternative engagement structures may be utilized according to aspects of the present disclosure.

Hand control 160 may be disposed on the proximal end 122 of the microcatheter 120. The hand control 160 may be able to extend and retract the engagement structure 142 from the microcatheter 120. The hand control 160 is single-handedly operable. The hand control 160 may include a brace 162 connected to the proximal end 122 of the microcatheter 120 and a push-pull mechanism 164 attached to the elongated member 144. The push-pull mechanism 164 may include a finger ring 164, but this is merely an example.

Figures 2A-2B illustrate example hand controls 270 for a combination stentriever and microcatheter according to aspects of the present disclosure. Hand control 270 may include a base 272 attached to the proximal end of a microcatheter 220 and a push-pull mechanism 274 attached to a proximal end of a stentriever. For example, push-pull mechanism 274 may include a pull-tab 274.

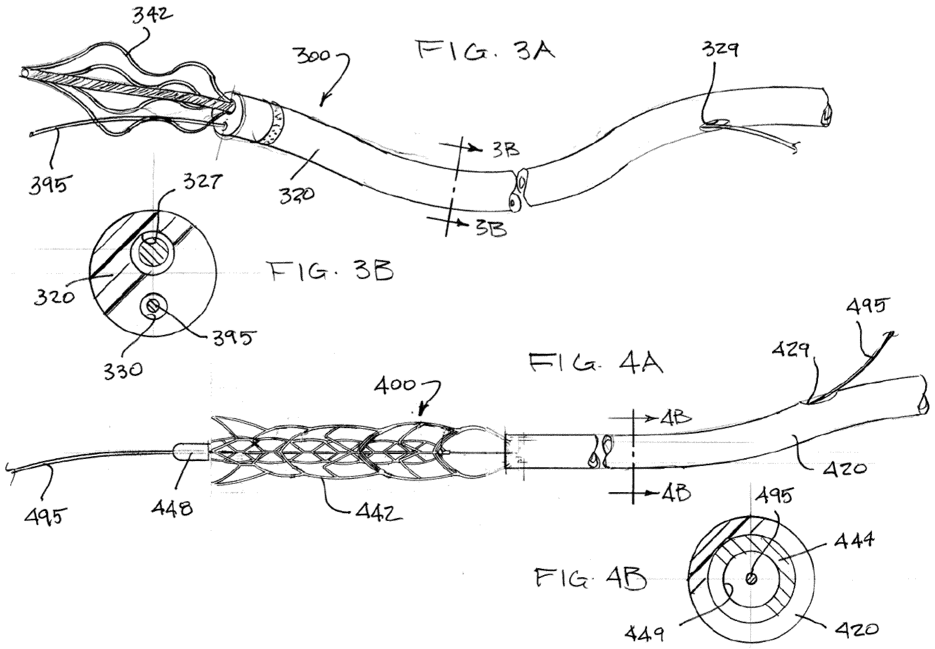

Figures 3A-5 illustrate example combination stentriever and microcatheters 300-500 according to aspects of the present disclosure. One of ordinary skill will recognize that the combination stentriever and microcatheters 300-500 may be substantially similar to the combination stentriever and microcatheter 100 described above with reference to Figures 1A and 1B, and/or aspects of combination stentriever and microcatheter 300-500 may be incorporated into combination stentriever and microcatheter 100.

Referring to Figure 3A, combination stentriever and microcatheter 300 may include a microcatheter 320 and a stentriever with an engagement structure 342. A cross-section of microcatheter 320 is illustrated in Figure 3B. Microcatheter 320 may include a lumen 327 for receiving the stentriever and a guide lumen 330 for receiving a guidewire 395. In some cases, microcatheter 320 may include a rapid exchange port 329, and the guide lumen 330 may only extend from the rapid exchange port 329 to a distal end of the microcatheter 320.

Referring to Figure 4A, combination stentriever and microcatheter 400 may include a microcatheter 420 and a stentriever with an elongated member an engagement structure 442 having a nose cone 448 and an elongated member 444 having a hollow or partially body 449. A cross-section of combination stentriever and microcatheter 400 is illustrated in Figure 4B. The hollow body 449 may be dimensioned to receive a guidewire 495. In some cases, microcatheter 420 may include a rapid exchange port 429. The rapid exchange port 429 may be dimensioned to receive a guidewire, and the guidewire may be fed through a stentriever port into the hollow interior of the stentriever and through a distal end of the stentriever. Nose cone 448 may be configured to drive through a clot (e.g., as a puncturing element).

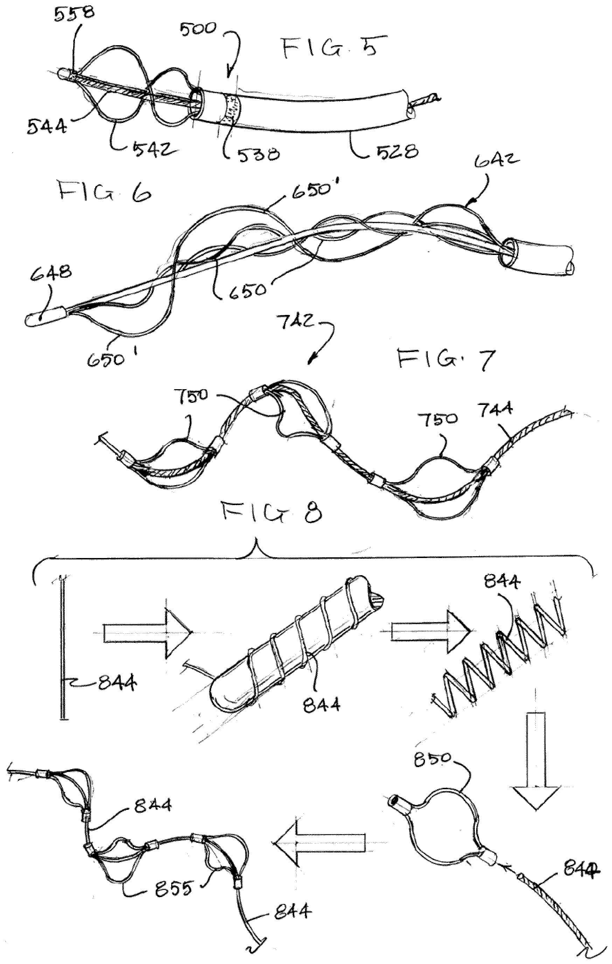

Referring to Figure 5, combination stentriever and microcatheter 500 may include a microcatheter having a first radioactive marker 538 disposed on its distal end. For example, radioactive marker 538 may be disposed on an outer surface 528 of the microcatheter. Combination stentriever and microcatheter 500 may also include a stentriever having an engagement structure 542 and an elongated member 544. A second radioactive marker 558 may be disposed on a distal end of the stentriever. By utilizing the first and second radioactive markers 538, 558, an operator may be able to more accurately determine a positioning and extend/retract status of combination stentriever and microcatheter 500.

Figures 6 and 7 illustrate example stentriever engagement structures according to aspects of the present disclosure. Referring to Figure 6, engagement structure 644 includes a nose cone 648 and a series of inner pinching cells 650 and outer pinching cells 650'. Referring to Figure 7, engagement structure 742 includes one or more pinching cells 750 with a shaped wire 744 threaded therethrough.

Figure 8 illustrates production of a stentriever according to aspects of the present disclosure. Referring to FIG. 8, a wire 844 is shaped by wrapping the wire around an object (e.g., a substantially cylindrical object). The shaped wire 844 is then threaded through and/or attached to one or more pinching cells 850. In this way, the engagement structure may be more readily developed than the processes utilized in the related art.

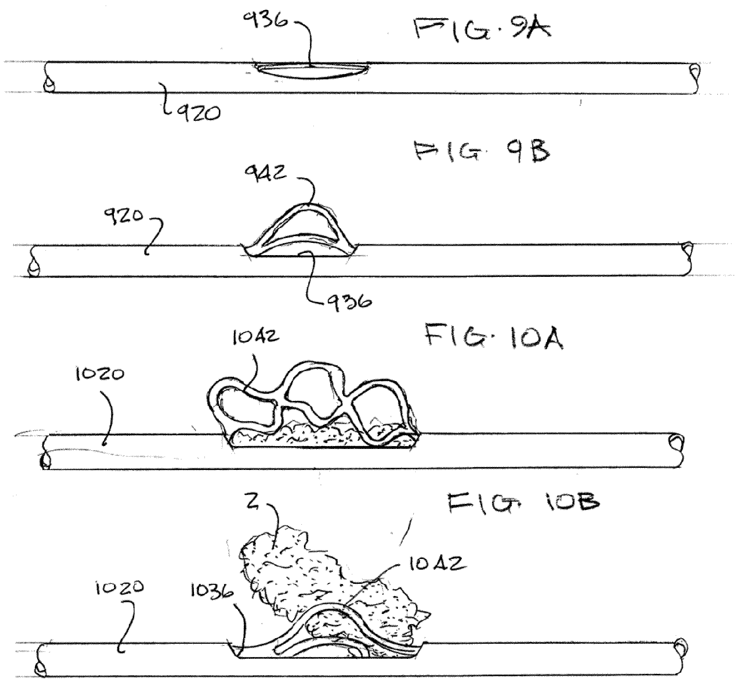

Figures 9A-10B illustrates combination stentriever and microcatheters with a side port according to aspects of the present disclosure. Referring to Figures 9A and 9B, microcatheter 920 includes a side port 936. Side port 936 may extend from an outer surface of the microcatheter to the lumen formed therein. An engagement structure 942 of a stentriever may be configured to extend and retract through the side port 936 (e.g., via operation of hand control 160). The engagement structure 942 may include a single pinching cell, but this is merely an example. In some cases, a distal end of the stentriever may be connected (e.g., permanently or semi-permanently) to an inner portion of the microcatheter 920.

Referring to Figures 10A and 10B, microcatheter 1020 includes a side port 1036. Side port 1036 may extend from an outer surface of the microcatheter to the lumen formed therein. An engagement structure 1042 of a stentriever may be configured to extend and retract through the side port 1036 (e.g., via operation of hand control 160) to engage with a clot 2. The engagement structure 1042 may include a plurality of pinching cells, but this is merely an example. In some cases, a distal end of the stentriever may be connected (e.g., permanently or semi-permanently) to an inner portion of the microcatheter 1020.

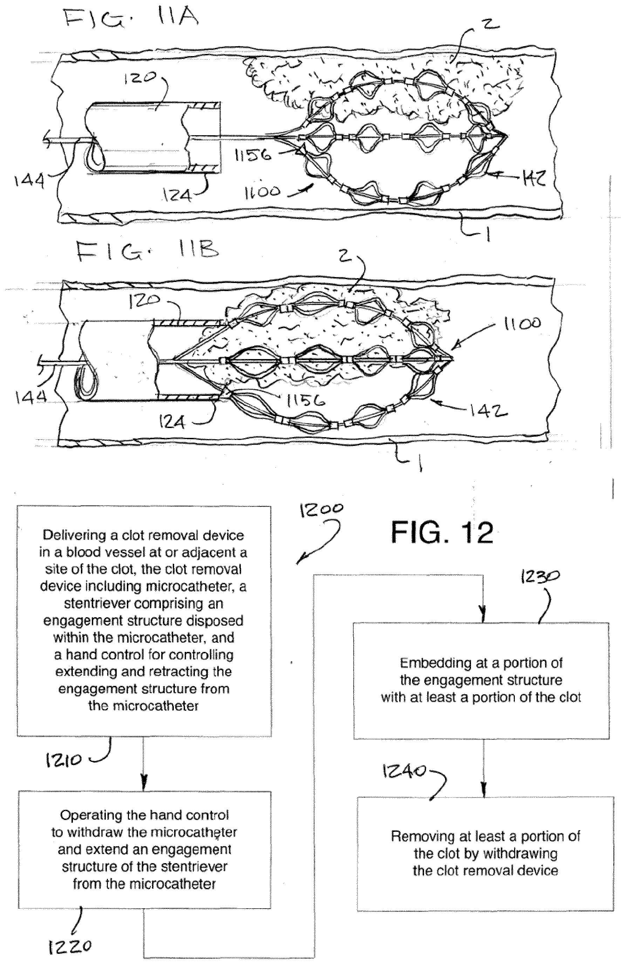

Figures 11A and 11B illustrate an operation of a combination microcatheter and stentriever 1100 having a stop 1156 according to aspects of the present disclosure. Microcatheter and stentriever 1100 may be moved within a blood vessel 1 to clot 2. The engagement structure 142 may be positioned within the microcatheter, e.g., in a collapsed configuration. Once positioned correctly, engagement structure 142 may be extended from microcatheter 120. The engagement structure 142 may interfere with clot 2. The engagement structure 142 may then be partially retracted into the microcatheter 120, for example, as shown in Figure 11B. For example, stentriever 140 may include a stop 1156 configured to prevent full retraction of the engagement structure 142 within the microcatheter 120 after the engagement structure 142 is extended. Accordingly, engagement structure 142 may be prevented from being overly retracted, and pinch clot 2 against microcatheter 120 (e.g., in a pinching configuration). One of ordinary skill, in light of the present disclosure, understands that stop 1156 is only one example to prevent over-extension. In some cases, the diameter of the engagement structure 142 in the expanded configuration may be greater than the diameter of the lumen such that the engagement structure 142 cannot be fully retrieved within the microcatheter 120 following deployment.

In some cases, stop 1156 may be retractable, for example, via operation of hand control 160 (e.g., via retrieval means). Accordingly, an operator may retract engagement structure 142 as needed if complete retraction or retraction beyond what is allowed by stop 1156.

Figure 12 is a flowchart 1200 of a treatment incorporating an example clot removal device (e.g., combination stentriever and microcatheter 100) according to aspects of the present disclosure. The method includes delivering 1210 a clot removal device in a blood vessel 1 (e.g., a body vessel) at or adjacent a site of the clot 2. The clot removal device may be a combination microcatheter and stentriever (e.g., 100) and may include a microcatheter (e.g., 120), a stentriever (e.g., 140), and a hand control (e.g., 160). The hand control 160 may be operated 1220 to withdraw the microcatheter 120 and extend an engagement structure 142 of the stentriever 140 from the microcatheter 120. The engagement structure may then embed 1230 within at least a portion of the clot 2. The stentriever and microcatheter 100 are then withdrawn, removing 1240 a portion of the clot 2. In some cases, the hand control 160 can be operated to at least partially retract the engagement structure 142 within the microcatheter 120 prior to withdrawing the microcatheter 120 and stentriever 140. In some cases, a stop or alternative structure may prevent over-retraction of stentriever 140.

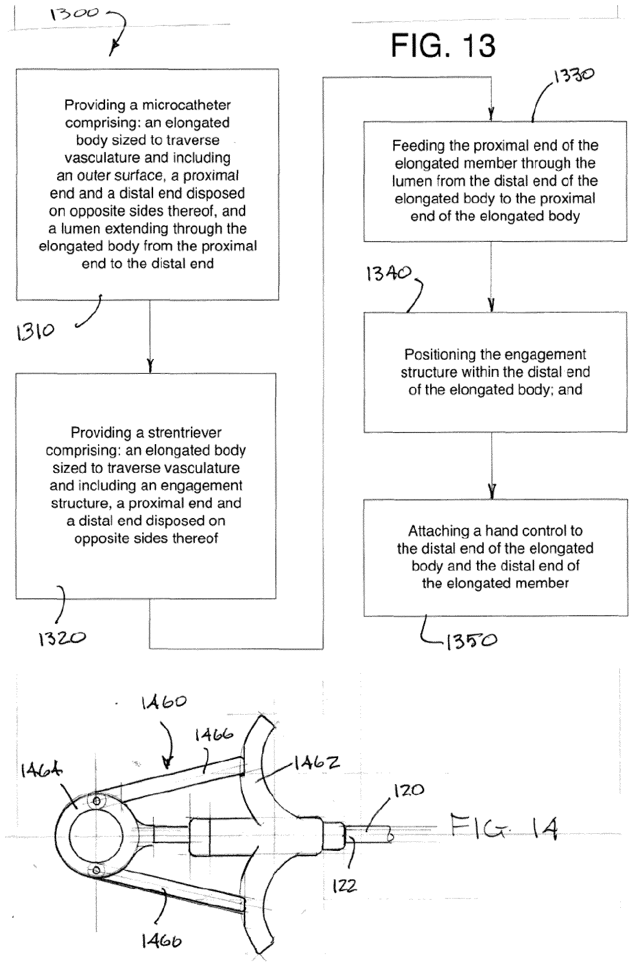

Figure 13 is a flowchart of producing a combination stentriever and microcatheter (e.g., 100) according to aspects of the present disclosure. There may be provided 1310 a microcatheter (e.g., 120). The microcatheter 120 may include an elongated body sized to traverse vasculature (e.g., 1) and including an outer surface (e.g., 18), a proximal end and a distal end disposed on opposite sides thereof, and a lumen (e.g., 127) extending through the elongated body from the proximal end to the distal end. The method may further include providing 1320 a stentriever (e.g., 140). The microcatheter may include an elongated body (e.g., 144) sized to traverse vasculature and an engagement structure (e.g., 142), a proximal end and a distal end disposed on opposite sides thereof, and a lumen extending through the elongated body from the proximal end to the distal end.

The method may further include feeding 1330 the proximal end of the elongated member through the lumen from the distal end of the elongated body to the proximal end of the elongated body. The engagement structure 142 may then be positioned 1340 within the distal end of the elongated body (e.g., 144 within lumen 127). Then a hand control (e.g., 160) may be attached 1350 to the distal end of the elongated body and the distal end of the elongated member.

Figure 14 illustrates a hand control 1460 with a lock 1466 according to aspects of the present disclosure. Lock 1466 may hold hand control 1460 in a substantially retracted position. For example, hand control 460 may include a brace 1462 and a push-pull mechanism 1464. Lock 1466 may prevent push-pull mechanism 1464 from moving forward and extending engagement structure 142.

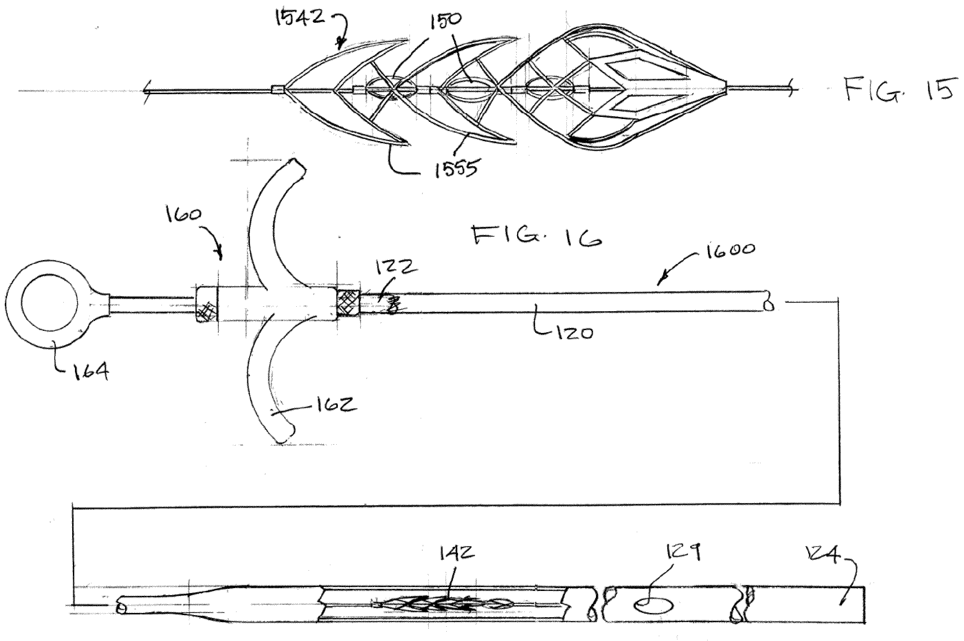

Figure 15 illustrates an engagement structure 1542 having scaffolding elements 1555 according to aspects of the present disclosure. The scaffolding elements 1555 may be configured to receive a clot (e.g., 2). In some cases, scaffolding elements 1555 may be combined with one or more pinching cells (e.g., 150).

Figure 16 illustrates a combination stentriever and microcatheter 1600 with an engagement structure 142 proximal to a rapid exchange port 129 according to aspects of the present disclosure.

The descriptions contained herein are examples of embodiments of the invention and are not intended in any way to limit the scope of the invention. As described herein, the invention contemplates many variations and modifications of the catheter 100 and methods for manufacturing and using the same. Additional modifications that are apparent to those having skill in the art to which this invention pertains and are intended to be within the scope of the claims which follow.

Aspect of the invention:
1. A method for removing a clot, comprising:
   operating a hand control to withdraw a microcatheter and extend an engagement structure of a stentriever from the microcatheter.

## Claims

1. A clot removal system for removing a clot from a body vessel, the clot removal system comprising:
a microcatheter comprising:
a distal segment comprising a first diameter; and
a proximal segment comprising a second diameter;
a stentriever positionable substantially within a lumen of a distal end of the microcatheter, the stentriever comprising:
an engagement structure configured to expand when distal of the microcatheter in an expanded configuration, and
an elongated member proximal of the engagement structure and extending to a proximal end of the microcatheter; and
a hand control disposed on the proximal end of the microcatheter for controlling extending and retracting the engagement structure from the microcatheter.

2. The clot removal system of claim 1, wherein the engagement structure is configured to be prevented from sliding proximal of the distal segment in a collapsed configuration.

3. The clot removal system of claim 1, wherein the hand control comprises:
(i) a brace connected to the proximal end of the microcatheter; and
a push-pull mechanism attached to the elongated member, or
(ii) a lock to hold the engagement structure in a substantially fully retracted position.

4. The clot removal system of claim 1, wherein the microcatheter further comprises a rapid exchange port extending from an outer surface of the microcatheter to the lumen, the rapid exchange port being dimensioned to receive a guidewire, the engagement structure being disposed within microcatheter proximal to the rapid exchange port.

5. The clot removal system of claim 1, wherein the microcatheter further comprises:
a rapid exchange port on an outer surface of microcatheter, the rapid exchange port being dimensioned to receive a guidewire; and
a guide lumen extending from the rapid exchange port to the distal end of the microcatheter.

6. The clot removal system of claim 1, wherein
microcatheter further comprises a rapid exchange port on an outer surface of the microcatheter,
the elongated member comprises a substantially hollow body extending through the engagement structure, and
the stentriever further comprises a stentriever port extending from an outer surface of the elongated member to an interior of the hollow body, the rapid exchange port, hollow body, and stentriever port being dimensioned to receive a guidewire, optionally wherein the stentriever port is proximal the rapid exchange port.

7. The clot removal system of claim 1, wherein the stentriever further comprises a nose cone on a distal end of the engagement structure.

8. The clot removal system of claim 1, wherein the lumen comprises:
a distal shaft extending proximally from the distal end, the distal shaft having a third diameter; and
a proximal shaft extending distally from the proximal end, the proximal shaft having a fourth diameter less than the third diameter, optionally wherein a diameter of the engagement structure in the expanded configuration is greater than the third diameter such that the engagement structure cannot be fully retrieved within the distal shaft.

9. The clot removal system of claim 1, wherein the microcatheter further comprises:
a proximal shaft extending distally from the proximal end; and
a distal shaft extending distally from the proximal shaft, the engagement structure being disposed within the distal shaft and being unable to be received within the proximal shaft.

10. The clot removal system of claim 1, wherein the microcatheter further comprises:
a proximal shaft extending distally from the proximal end, the lumen comprising a first diameter through the proximal shaft; and
a distal shaft extending distally from the proximal shaft, the lumen comprising a second diameter through the distal shaft, the first diameter being less than the second diameter such that the engagement structure cannot be received by the lumen in the proximal shaft.

11. The clot removal system of claim 1, wherein the microcatheter further comprises:
a distal shaft extending proximally from the distal end; and
a side port extending from an outer surface of the microcatheter at the distal shaft to the lumen,
wherein the engagement structure is disposed within the distal shaft proximate the side port, the hand control is configured to control extending and retracting the engagement structure from the side port, optionally wherein a distal end of the engagement structure is attached to the microcatheter.

12. The clot removal system of claim 1, wherein the engagement structure comprises at least one stop configured to prevent full retraction of the engagement structure within the lumen after extension.

13. The clot removal system of claim 12, wherein the at least one stop prevents retraction of the engagement structure beyond a pinching configuration.

14. The clot removal system of claim 13, wherein the hand control further comprises a retrieval means to control withdrawing the at least one stop to enable full retraction of the engagement structure.

15. The clot removal system of claim 1 further comprising:
a first radioactive marker disposed on the distal end of the elongated member; and
a second radioactive marker disposed on a distal end of the engagement structure.
